# EUROPEAN PATENT SPECIFICATION

(11) **EP 3 528 804 B1**
(45) Date of publication and mention of the grant of the patent: **26.07.2023**
(21) Application number: 17863114.9
(22) Date of filing: 18.10.2017
(51) Int. Cl.: A61K 31/352, A61K 9/68, A61K 31/485, A61K 31/05, A61K 45/06, A61P 25/36

(54) **CHEWING GUM COMPOSITION COMPRISING CANNABINOIDS AND OPIOID AGONISTS AND/OR ANTAGONISTS**
KAUGUMMIZUSAMMENSETZUNG MIT CANNABINOIDEN UND OPIOID-AGONISTEN UND/ODER -ANTAGONISTEN
COMPOSITION DE GOMME À MÂCHER COMPRENANT DES CANNABINOÏDES ET DES AGONISTES ET/OU DES ANTAGONISTES OPIOÏDES

(30) Priority: 20.10.2016 US 201662410469 P
(43) Date of publication of application: 28.08.2019
(73) Proprietor: APIRX Pharmaceutical USA, LLC, New York NY 10022 (US)
(72) Inventor: ANASTASSOV, George, Glen Cove, New York 11542 (US); CHANGOER, Lekhram, 3404 HL lJsselstein (NL)
(74) Representative: Nederlandsch Octrooibureau
(86) International application number: PCT/US2017/057229
(87) International publication number: WO 2018/075665

(56) References cited:
- WO-A2-2008/024490
- CA-A1- 2 937 471
- US-A1- 2002 160 043
- US-A1- 2009 246 265
- US-A1- 2015 209 322

## Description

### CROSS-REFERENCE TO RELATED APPLICATIONS

This application claims priority to United States Provisional Application No. 62/410,469, filed October 20, 2016.

### BACKGROUND OF THE INVENTION

### Field of the Invention

Opioid addiction treatment and cannabis dependence treatment are developing areas with many proposed techniques and remedies. This invention involves a chewing gum composition with controlled release of cannabinoids and opioid agonists and/or antagonists for opioid and cannabis addiction and/or dependence treatment. This chewing gum may also be used for treatment of chronic pain.

### Description of the Related Technology

Opioids are a group of analgesic agents commonly used in clinical practice but are also commonly seen as addictive agents. Opioids bind to opioid receptors, which are found in the central and peripheral nervous system and the gastrointestinal tract. These receptors mediate both psychoactive and the somatic effects of opioids.

Opioids agonists include morphine, codeine, thebaine, hydrocodone, hydromorphone, oxycodone, oxymorphone, buprenorphine, fentanyl, methadone, pethidine, levorphanol, tramadol, and dextropropoxyphene.

Opioids cause euphoria and thus are used illicitly. In 2011, an estimated of 4 million people in the United States used opioids recreationally and were dependent on them. Opioid dependency may start with prescription use of opioid which turns into illicit drug use.

Physical dependence is the physiological adaptation of the body to the presence of a substance, in this case an opioid. It is the development of withdrawal symptoms when the substance is discontinued. Withdrawal symptoms of opiates may include severe dysphoria, craving, irritability, sweating, nausea, tremor, vomiting, and myalgia.

The speed and severity of withdrawal symptoms occurrence depend on half-life of the opioid. Heroin and morphine withdrawal occur more quickly and are more severe than methadone withdrawal. The acute withdrawal phase is often followed by a protracted phase of depression and insomnia that can last for months.

Addiction is marked by a change in behavior caused by the biochemical changes in the brain after continued substance abuse. Substance use becomes the main priority of the addict, regardless of the harm they may cause to themselves or others. An addiction causes people to act irrationally when they don't have the substance they are addicted to in their system. Addiction encompasses both mental and physical reliance on the substance.

Opioid addiction therapy depends on a variety of techniques. Among them is replacement therapy, wherein an opioid is replaced with another less addictive opioid, which curbs the craving feeling and reduces withdrawal symptoms, while maintains the subject's mental state such that the subject is still able to function normally.

Opioid replacement therapy is not without adverse effect. Methadone used long term may lead to potentially deadly slowed breathing. The analgesic effect in methadone ends long before the methadone half life, thus more methadone is needed, causing a built up of methadone.

The cannabis plant has many naturally occurring substances that are of great interest in the fields of science and medicine. Isolated compounds from the cannabis plant include Δ⁹-tetrahydrocannabinol (THC), cannabidiol (CBD), cannabichromene (CBC), cannabigerol (CBG), cannabidivarin (CBDV), among other compounds. While THC has psychoactive effects, CBD, CBC, CBG, and CBDV do not. Isolated compounds from the cannabis plant are called cannabinoids. There are a total of one hundred and forty one (141) cannabinoids that have been isolated from the cannabis plant. Many researchers have confirmed the medicinal value of cannabinoids. Cannabinoids have been investigated for possible treatment of seizures, nausea, vomiting, lack of appetite, pain, arthritis, inflammation, and other conditions.

The IUPAC nomenclature of THC is (-)-(6aR,10aR)-6,6,9-trimethyl-3-pentyl-6a,7,8,10a-tetrahydro-6H-benzo[c]chromen-1-ol. CBD's IUPAC nomenclature is 2-((1S,6S)-3-methyl-6-(prop-1-en-2-yl)cyclo-hex-2-enyl)-5-pentylbenzene-1,3-diol). CBC has the IUPAC nomenclature of 2-methyl-2-(4-methylpent-3-enyl)-7pentyl-5-chromenol. These are among the most prominent compounds in the family of compounds extracted from the cannabis plant referred to as cannabinoids.

Cannabinoids may be isolated by extraction or cold pressing of cannabis plants. Plants in the cannabis genus include *Cannabis saliva. Cannabis ruderalis,* and *Cannabis indica.* These plants are the natural sources of cannabinoids. Cannabinoids are also available in synthetic forms. Methods to synthesize cannabinoids in lab settings were discovered and are still currently practiced. Synthetic cannabinoids are more targeted, in that the synthetic compound usually comes isolated without other cannabinoids mixed in.

Cannabinoids from industrial hemp are marketed in the United States, such as cannabidiol. Various products containing cannabinoids have been marketed in recent years. Cannabinoids may be consumed by ingestion, by inhalation, via transmucosal, or by transdermal delivery. Patent document CA2937471 discloses chewing gum formulations comprising cannabinoid.

Cannabis dependence is mainly due to Δ⁹-THC presence in cannabis. When a mammal consumes cannabis, Δ⁹-THC gives the "high" feeling. Cannabis addiction in the form of smoking cannabis also gives rise to lung cancer risk similar to tobacco smoking.

Opioid addiction treatment remains a challenge. This invention proposes a product to treat opioid addiction standing alone or concurrent opioid and Δ⁹-THC addiction/dependence.

### ABBREVIATIONS

CBC: cannabichromene
CBD: cannabidiol
CBDV: cannabidivarin
CBG: cannabigerol
Δ⁹-THC: delta-9-tetrahydrocannabinol

### SUMMARY

The present invention relates to a chewing gum composition according to the claims comprising cannabinoids or derivatives thereof and at least one opioid agonist and/or antagonist. Cannabinoids or derivatives thereof and opioid agonists and/or antagonists are under controlled release during mastication and consumption. Also disclosed, but not claimed, is the use of this chewing gum composition in treating opioid addiction or dependence, concurrent cannabis and opioid addiction and/or dependence, or pain.

This invention provides a chewing gum composition comprising, based on total weight of the composition: 0.1 to 5% by weight of at least one cannabinoid; 0.01 to 1% by weight of at least one opioid; at least 20% by weight of a gum base; 5 to 35% by weight of at least one buffering agent selected from the group consisting of acetates, glycinates, phosphates, carbonates, glycerophosphates, citrates, and borates; 1 to 10% by weight of at least one flavoring agent selected from the group consisting of peppermint, spearmint, licorice, cinnamon, watermelon, vanilla, pineapple, apple, and cranberry; 1 to 65% by weight of at least one sweetening agent selected from the group consisting of isomalt, sorbitol, stevia, maltitol, and xylitol; and at least one anti-oxidant selected from the group consisting of ascorbyl palminate and sodium ascorbate, wherein the at least one cannabinoid is provided in microencapsulated form, nanoencapsulated form, or in freeze dried form.

This invention provides a chewing gum composition according to embodiments wherein the at least one cannabinoid is cannabidiol, Δ⁹-tetrahydrocannabinol, cannabichromene, cannabigerol, cannabidivarin, derivatives thereof, or their acid metabolites.

This invention provides a chewing gum composition according to embodiments wherein the at least one cannabinoid is provided in combination with at least one suitable carrier selected from the group consisting of sugar alcohol, microcrystalline cellulose derivatives, dextran, agarose, agar, pectin, alginate, xanthan, chitosan, and starch.

This invention provides a chewing gum composition according to embodiments wherein the at least one cannabinoid is provided in microencapsulated form, nanoencapsulated form, or in freeze dried form.

This invention provides a chewing gum composition according to embodiments wherein the at least one cannabinoid is provided in internal voids within a suitable solid carrier.

This invention provides a chewing gum composition according to embodiments wherein the at least one cannabinoid is provided in a granule within the gum matrix.

This invention provides a chewing gum composition according to embodiments wherein the at least one cannabinoid is procured from natural sources, such as deriving or extracting from cannabis plants, or synthetic.

This invention provides a chewing gum composition according to embodiments wherein the at least one opioid is an opioid agonist selected from the group consisting of morphine, codeine, thebaine, hydrocodone, hydromorphone, oxycodone, oxymorphone, buprenorphine, fentanyl, methadone, pethidine, levorphanol, tramadol, and dextropropoxyphene.

This invention provides a chewing gum composition according to embodiments wherein the at least one opioid is an opioid prodrug selected from the group consisting of 6-monoacetyl morphine, nicomorphine, dipropanoylmorphine, desomorphine, methyldesorphine, acetylpropionyl morphine, dibenzoyl morphine, and diacetyl dihydromorphine.

This invention provides a chewing gum composition according to embodiments wherein the at least one opioid is an opioid antagonist selected from the group consisting of Naloxone hydrochloride dehydrate and natrexone hydrochloride.

This invention provides a chewing gum composition according to embodiments wherein the at least one opioid comprises both opioid agonist and opioid antagonist.

This invention provides a chewing gum composition according to embodiments wherein the at least one opioid is provided in nanoencapsulated or microencapsulated form.

This invention provides a chewing gum composition according to embodiments wherein the preservative is citric acid.

The invention provides a chewing gum composition according to embodiments which may further comprise at least one pharmaceutically acceptable excipient selected from the group consisting of fillers, disintegrants, binders, and lubricants.

This invention provides a chewing gum composition according to embodiments which may further comprise silicon dioxide or magnesium stearate.

Also disclosed, but not claimed, is a use of cannabinoids and opioids for the manufacture of a cannabinoid and opioid chewing gum composition for treatment of opioid addiction or dependence according to this invention.

Also disclosed, but not claimed, is a use of cannabinoids and opioids for the manufacture of a cannabinoid and opioid chewing gum composition for treatment of opioid addiction or dependence in a mammal in need thereof, comprising administering to the mammal a chewing gum composition according to this invention, wherein the mammal receives the chewing gum administration 1 to 6 times a day.

Also disclosed, but not claimed, is a use of cannabinoids and opioids for the manufacture of a cannabinoid and opioid chewing gum composition for treatment of opioid addiction or dependence concurrent with cannabis addiction or dependence in a mammal in need thereof, comprising administering to the mammal a chewing gum composition according to this invention.

Also disclosed, but not claimed, is a use of cannabinoids and opioids for the manufacture of a cannabinoid and opioid chewing gum composition for treatment of opioid addiction concurrent with cannabis dependence in a mammal in need thereof, comprising administering to the mammal a chewing gum composition according to this invention, wherein the mammal receives the chewing gum administration 1 to 6 times a day.

Also disclosed, but not claimed, is a use of cannabinoids and opioids for the manufacture of a cannabinoid and opioid chewing gum composition for treatment of pain in a mammal in need thereof, comprising administering to the mammal a chewing gum composition according to this invention.

### DETAILED DESCRIPTION OF CERTAIN INVENTIVE EMBODIMENTS

Embodiments of this application relate to a chewing gum composition comprising cannabinoids and opioid agonists and/or antagonists, wherein cannabinoids and opioid agonists and/or antagonists are incorporated into the chewing gum for controlled release. The chewing gum composition may be consumed by a human for cessation of opioid and/or cannabis dependence and/or addiction.

In embodiments, the chewing composition may comprise 0.1 - 5% by weight of at least one cannabinoid or derivatives thereof based on total weight of the composition. In a 2g chewing gum piece, cannabinoids or derivatives thereof may comprise 2 - 100 mg. Cannabinoids in the chewing gum composition according to embodiments may be synthetic or procured from natural source.

Cannabinoids may also be in oily form, as cannabis oil, hemp oil, or hashish oil. In these embodiments, cannabinoids may be provided in a carrier to prevent absorption into the gum matrix. It is contemplated that cannabinoids provided in oily form may be used in the embodiments described herein.

Cannabinoids may be provided in a solid material carrier composed of an edible solid, such as a sugar alcohol, and cannabinoids, to prevent binding with the gum base. Cannabinoids may be embedded into the sugar alcohol. Other solids suitable for embedding cannabinoids are contemplated, such that cannabinoids are provided within internal voids of solid materials. Alternatively, cannabinoids or derivatives thereof may be provided in a granule embedded into the gum matrix. Cannabinoids provided in these manners may improve cannabinoid release during mastication of the chewing gum according to embodiments.

Cannabinoids are provided in microencapsulated or nanoencapsulated form or in freeze dried form. Microencapsulated or nanoencapsulated and freeze dried cannabinoids may improve the chewing gum's taste, improve stability, prevent binding with the gum matrix, control cannabinoid release during mastication, and further improve bioavailability of the cannabinoids once entering the gastrointestinal tract.

In embodiments, cannabinoids provided in encapsulated form may be particles of size 20-40 nm, which may improve bioavailability profiles of cannabinoids and prevent degradation in gastric fluid. Encapsulation may result in liposomal particles containing cannabinoids and derivatives thereof.

In these embodiments, cannabinoids may be Δ⁹-tetrahydrocannabinol (THC), cannabichromene (CBC), cannabigerol (CBG), cannabidivarin (CBDV), cannabidiol (CBD), other cannabinoids, derivatives thereof, their acid metabolites, or a combination of cannabinoids and/or their acid metabolites and/or derivatives thereof. Cannabinoids described herein may be natural or synthetic cannabinoids.

Other suitable carriers which may be combined with cannabinoids before inclusion into the gum matrix may include certain celluloses, such as microcrystalline cellulose derivatives, dextran, agarose, agar, pectin, alginate, xanthan, chitosan, or starch. The combination of cannabinoids and suitable carriers may result in cannabinoid being present within internal voids of these carriers.

Providing cannabinoids by combining with a suitable carrier or by providing cannabinoids in a capsule within the gum matrix may enable controlled release of cannabinoids during chewing of the chewing gum composition. Providing cannabinoids in microencapsulated, nanoencapsulated, or freeze dried form may also enable controlled release of cannabinoids during chewing of the chewing gum composition.

In embodiments, the chewing gum composition comprises at least one opioid to act as opioid agonist and/or antagonist. Opioids are present in the chewing gum composition at 0.01 - 1% by weight based on the total weight composition. In a 2g chewing gum piece, opioid may comprise 0.2 - 20 mg.

Opioids in the chewing gum composition according to embodiments may be opioid agonists. Opioid agonists may be derived from plant, such as morphine, codeine, or thebaine. Semi-synthetic opioids may be used, such as hydrocodone, hydromorphone, oxycodone, oxymorphone, or buprenorphine. Buprenorphine, in particular, is both an opioid agonist and antagonist, and thus its effect in opioid addiction in treatment is both as a replacement opioid and an antagonist of opioids. Synthetic opioids may also be used, such as fentanyl, methadone, pethidine, levorphanol, tramadol, or dextropropoxyphene. Prodrugs of the above opioids may also be used, such as 6-monoacetyl morphine, nicomorphine (morphine dinicotinate), dipropanoylmorphine (morphine dipropionate), desomorphine (di-hydro-desoxy morphine), methyldesorphine, acetylpropionyl morphine, dibenzoyl morphine, or diacetyl dihydromorphine.

Opioid antagonists such as Naloxone hydrochloride dehydrate (C₁₉H₂₁NO₄) and naltrexone hydrochloride (C₂₀H₂₃NO₄) may also be included in the chewing gum composition according to embodiments in addition to opioid agonists. Where opioid antagonists are included, opioid antagonists may be at 3:1 to 5:1, more specifically 4:1 ratio by weight for opioid agonist: opioid antagonist.

Certain opioids may reduce craving sensation while minimizing adverse effects on users. In opioid addiction treatments, withdrawal symptoms may be the main obstacle to recovery. When opioid agonists are provided, they may bind to opioid receptors, reducing the adverse effect of opioid withdrawal. Moreover, opioids consumed by injection, such as heroin injection, may pose additional risks to users. By providing replacement opioids in a controlled release chewing gum, replacement opioids may curb craving sensation while reducing adverse withdrawal symptoms and preventing adverse effects caused by injection. On the other hand, adding an opioid antagonist may counteract the effects of the agonists, for example the addictive opioid such as heroin, and thus eventually reduce opioid addiction.

Cannabinoids, on the other hand, may also curb craving sensation. Finally, by providing replacement opioids such as opioid agonists and/or antagonists and cannabinoids in a chewing gum form, users may avoid adverse effects caused by injection and/or smoking.

In embodiments, opioids provided may be microencapsulated or nanoencapsulated. Encapsulation may improve the chewing gum's taste as a whole. Encapsulation may aid with dissolution in the subject's oral cavity and transmucosal delivery mechanism. Encapsulation may also enable controlled release of opioids during chewing of the chewing gum composition. Encapsulation may improve opioids' bioavailability profile upon mastication of the chewing gum composition.

Methods to encapsulate opioids may be methods commonly used in the art, such as precision particle encapsulation. A method to encapsulate active ingredients in described in Berkland, C., M. King, et al. (2002). "Precise control of PLG microsphere size provides enhanced control of drug release rate." J Control Release 82(1): 137-147.

Gum base provided for the chewing gun composition according to these embodiments may be non-disintegrating. Gum base such as Gum powder PG 11 TA, Gum powder PG 11 TA New, Gum powder PG 5 TA, Gum powder PG 5 TA New, and Gum powder PG N12 TA may be used. Gum base is present at least 20% by weight of the composition.

In embodiments, at least one buffering agent may be included in this chewing gum composition. Suitable buffering agents may include acetates, glycinates, phosphates, carbonates, glycerophosphates, citrates, borates, and/or mixtures thereof. Buffering agents are present at 5 - 35% by weight.

In embodiments, the chewing gum composition may have other ingredients to improve organoleptic properties. The chewing gum composition according to embodiments includes at least one flavoring agent and at least one sweetening agent.

Ingredients such as certain flavoring agents are included. Flavoring agents may include peppermint, spearmint, licorice, cinnamon, watermelon, vanilla, pineapple, apple, cranberry, and/or other suitable flavoring agents. Certain food colorants may be included to improve the aesthetic appearance of the chewing gum composition. Flavoring agents are present in this chewing composition at 1 - 10% by weight.

Sweetening agents are present at 1 - 65% of by weight of the composition according to embodiments. Sweetening agents used in chewing gums according to embodiments may be isomalt, sorbitol, stevia, maltitol, xylitol, or other suitable sweetening agents, and/or combinations thereof.

In embodiments, the chewing gum composition may comprise ingredients for preservation such as citric acid. Additional ingredient to assist with powder flow and prevent the gum base from sticking to manufacturing surfaces may be included. Such ingredient may be silicon dioxide or magnesium stearate. Other ingredients for preservation and manufacturing management may also be used.

Additional pharmaceutically acceptable excipients used in the chewing gum composition according to embodiments may be fillers, disintegrants, binders, or lubricants. Antioxidants such as ascorbyl palminate and sodium ascorbate are included. The chewing gum composition according to embodiments comprises at least one pharmaceutically acceptable excipient and/or at least one anti-oxidant.

The chewing gum composition according to embodiments may be made by a compressing process or by a hot process. In the compressing process, ingredients are mixed and compressed into the gum base using a compress machine. In the hot process, ingredients are mixed and heated before the gum base is poured in. The gum mixture is then molded and left to cure.

The gum mixture may then be cut into appropriate size for consumption, such as 2 grams for each piece. The gum pieces may be coated with a polyol coating such as sorbitol, maltitol, isomalt, or starch. The coating layer may prevent moisture from penetrating into the gum matrix. The gum pieces may be wrapped in separate pieces of wrapping materials and packaged into a pack or box, or packaged into a blister package.

The chewing gum composition, although not claimed, may be used for opioid dependence and/or addiction treatment. Opioid dependence and/or addiction may be treated or alleviated by consumption of chewing gums according to embodiments. Cannabinoids and opioid agonists and/ antagonists in these chewing gums may be released in a controlled manner and absorbed by a subject via transmucosal delivery mechanism. A mammal, such as a human being, may chew the chewing gum composition according to embodiments 1 - 6 times a day to aid with opioid craving sensation whiling curbing this craving.

The chewing gum composition, although not claimed, may be used in treatment of cannabis dependence, in particular Δ⁹-tetrahydrocannabinol dependence, concurrent with opioid addiction. Cannabis dependence, in particular Δ⁹-THC dependence, concurrent with opioid addiction may be treated or alleviated by consumption of chewing gums according to embodiments. A mammal, such as a human being, may chew the chewing gum composition according to embodiments 1 - 6 times a day to aid opioid and/or Δ⁹-THC craving sensation while curbing this craving.

The chewing gum composition, although not claimed, may be used in treatment of pain and/or chronic pain. A mammal, such as a human being, may chew the chewing gum composition according to embodiments as need for treatment of pain. A mammal, such as a human being, may chew the chewing gum composition according to embodiments 1 - 6 times a day to treat and/or alleviate pain.

### EXAMPLES

### Example 1

### Chewing gum composition preparation

Chewing gum compositions having 10mg of CBD and 1 mg of buprenorphine are prepared by cold pressing. Percentages are given in weight percentage.

**Table 1**

| **Phase** | **Raw material** | **Percentage (%)** |
|---|---|---|
| A1 | Isomalt | 28.89 |
| A2 | CBD (microencapsulated) | 0.50 |
| A3 | Cellulose | 0.50 |
| A4 | Buprenorphine (microencapsulated) | 0.04 |
| A5 | Naltrexone hydrochloride (microencapsulated) | 0.01 |
| B1 | Gum base | 24.50 |
| B2 | Sorbitol | 10.00 |
| B3 | Maltitol | 10.00 |
| B4 | Citric acid | 0.50 |
| B5 | Magnesium stearate | 2.00 |
| B6 | Silicon dioxide | 0.40 |
| B7 | Xylitol | 13.60 |
| B8 | Stevia | 1.05 |
| B9 | Vanilla | 4.00 |
| B10 | Spearmint/peppermint | 4.00 |
| B11 | Colorants FD&C blue | 0.01 |
| | **Total** | **100.00** |

Step 1: Make a blend of A2, A3, A4, and A5 into A1 to form Phase 1.
Step 2: Mix B1 - B 11 in a separate vessel to form Phase 2.
Step 3: Use a double layer chewing gum machine to compress Phase 1 and Phase 2 together.

Chewing gum composition may be cut into 2 gram pieces as appropriate. Chewing gum compositions with a mass at about 2 grams and containing 10 mg of CBD and 1 mg of buprenorphine were prepared.

### Example 2

### Chewing gum composition preparation

Chewing gum compositions having 10mg of Δ⁹-THC and 1 mg of buprenorphine are prepared. Percentages are given in weight percentage.

**Table 2**

| **Phase** | **Raw material** | **Percentage (%)** |
|---|---|---|
| A1 | Gum base | 75.00 |
| A2 | Xylitol | 14.00 |
| A3 | Glycerine | 4.50 |
| B1 | Sacharrine | 0.40 |
| B2 | Water | 1.50 |
| B3 | Buprenorphine (microencapsulated) | 0.05 |
| B4 | Citric acid | 0.50 |
| C1 | Peppermint aroma oil | 1.50 |
| A4 | Vanilla/cranberry flavor | 1.50 |
| C2 | Δ⁹-THC (microencapsulated) | 0.50 |
| A5 | Cellulose | 0.50 |
| | **Total** | **100.00** |

Step 1: Heat the gum base (A1) to 90 ⁰C, then add A2 - A5 to form Phase 1.
Step 2: Dissolve B1 and B4 in B2 to form Phase 2.
Step 3: Heat the peppermint oil (C1) to 60 - 70 ⁰C, then add C2 and B3 to form Phase 3.
Step 4: Add Phase 2 to Phase 1, stir vigorously and add Phase 3, stir for 7 minutes.
Step 5: Pour the gum mixture out and prepare chewing gum tablets by molding as need.

Chewing gum composition may be cut into 2 gram pieces as appropriate. Chewing gums with a mass at about 2 grams and containing 10 mg of Δ⁹-THC and 1 mg of buprenorphine were prepared.

### Example 3

### Chewing gum composition preparation

Chewing gums having 10 mg of CBD, 0.8 mg of thebaine, and 0.2 mg of Naloxone hydrochloride are prepared. Percentages are given in weight percentage.

**Table 3**

| **Phase** | **Raw material** | **Percentage (%)** |
|---|---|---|
| A1 | Isomalt | 28.89 |
| A2 | CBD (microencapsulated) | 0.50 |
| A3 | Cellulose | 0.50 |
| A4 | Thebaine (microencapsulated) | 0.04 |
| A5 | Naloxone hydrochloride (microencapsulated) | 0.01 |
| B1 | Gum base | 24.50 |
| B2 | Sorbitol | 10.00 |
| B3 | Maltitol | 10.00 |
| B4 | Citric acid | 0.50 |
| B5 | Magnesium stearate | 2.00 |
| B6 | Silicon dioxide | 0.40 |
| B7 | Xylitol | 13.60 |
| B8 | Stevia | 1.05 |
| B9 | Licorice | 4.00 |
| B10 | Spearmint | 4.00 |
| B11 | Colorants FD&C blue | 0.01 |
| | **Total** | **100.00** |

Step 1: Make a blend of A2, A3, A4, and A5 into A1 to form Phase 1.
Step 2: Mix B1 - B11 in a separate vessel to form Phase 2.
Step 3: Use a double layer chewing gum machine to compress Phase 1 and Phase 2 together.

Chewing gum composition may be cut into 2 gram pieces as appropriate. Chewing gums with a mass at about 2 grams and containing 10 mg of CBD, 0.8 mg of thebaine (semi-synthetic), and 0.2 mg of Naloxone hydrochloride were prepared.

## Claims

1. A chewing gum composition comprising, based on total weight of the composition:
0.1 to 5% by weight of at least one cannabinoid;
0.01 to 1% by weight of at least one opioid;
at least 20% by weight of a gum base;
5 to 35% by weight of at least one buffering agent selected from the group consisting of acetates, glycinates, phosphates, carbonates, glycerophosphates, citrates, and borates;
1 to 10% by weight of at least one flavoring agent selected from the group consisting of peppermint, spearmint, licorice, cinnamon, watermelon, vanilla, pineapple, apple, and cranberry;
1 to 65% by weight of at least one sweetening agent selected from the group consisting of isomalt, sorbitol, stevia, maltitol, and xylitol; and
at least one anti-oxidant selected from the group consisting of ascorbyl palminate and sodium ascorbate,
wherein the at least one cannabinoid is provided in microencapsulated form, nanoencapsulated form, or in freeze dried form.

2. The chewing gum composition of Claim 1, wherein the at least one cannabinoid is cannabidiol, Δ⁹-tetrahydrocannabinol, cannabichromene, cannabigerol, cannabidivarin, or their acid metabolites.

3. The chewing gum composition of Claim 1, wherein the at least one cannabinoid is provided in combination with at least one suitable carrier selected from the group consisting of sugar alcohol, celluloses, such as microcrystalline cellulose derivatives, dextran, agarose, agar, pectin, alginate, xanthan, chitosan, and starch.

4. The chewing gum composition of Claim 1, wherein the at least one cannabinoid is provided in internal voids within a suitable solid carrier.

5. The chewing gum composition of Claim 1, wherein the at least one cannabinoid is provided in a granule within the gum matrix.

6. The chewing gum composition of Claim 1, wherein the at least one cannabinoid is procured from natural sources or synthetic.

7. The chewing gum composition of Claim 1, wherein the at least one opioid is an opioid agonist selected from the group consisting of morphine, codeine, thebaine, hydrocodone, hydromorphone, oxycodone, oxymorphone, buprenorphine, fentanyl, methadone, pethidine, levorphanol, tramadol, and dextropropoxyphene.

8. The chewing gum composition of Claim 1, wherein the at least one opioid is an opioid prodrug selected from the group consisting of 6-monoacetyl morphine, nicomorphine, dipropanoylmorphine, desomorphine, methyldesorphine, acetylpropionyl morphine, dibenzoyl morphine, and diacetyl dihydromorphine.

9. The chewing gum composition of Claim 1, wherein the at least one opioid is provided in nanoencapsulated or microencapsulated form.

10. The chewing gum composition of Claim 1, further comprising at least one preservative and at least one antioxidant.

11. The chewing gum composition of Claim 10, wherein the preservative is citric acid.

12. The chewing gum composition of Claim 10, further comprising at least one pharmaceutically acceptable excipient selected from the group consisting of fillers, disintegrants, binders, and lubricants.

13. The chewing gum composition of Claim 12, further comprising silicon dioxide or magnesium stearate.

14. The chewing gum composition of Claim 1, further comprising at least one opioid antagonist selected from the group comprising of naloxone hydrochloride dehydrate and naltrexone hydrochloride at a weight ratio of 4 opioid : 1 opioid antagonist.

## Patentansprüche

1. Kaugummizusammensetzung, die bezogen auf das Gesamtgewicht der Zusammensetzung Folgendes umfasst:
0,1 bis 5 Gew.-% mindestens eines Cannabinoids;
0,01 bis 1 Gew.-% mindestens eines Opioids;
mindestens 20 Gew.-% einer Gummibasis;
5 bis 35 Gew.-% mindestens eines Puffermittels, das aus der Gruppe ausgewählt ist, die aus Azetaten, Glycinaten, Phosphaten, Karbonaten, Glycerophosphaten, Zitraten und Boraten besteht;
1 bis 10 Gew.-% mindestens eines Aromastoffs, der aus der Gruppe ausgewählt ist, die aus Pfefferminze, grüner Minze, Lakritz, Zimt, Wassermelone, Vanille, Ananas, Apfel und Preiselbeere besteht;
1 bis 65 Gew.-% mindestens eines Süßstoffs, der aus der Gruppe ausgewählt ist, die aus Isomalt, Sorbit, Stevia, Maltit und Xylit besteht; und
mindestens ein Antioxidans, das aus der Gruppe ausgewählt ist, die aus Ascorbylpalminat und Natriumascorbat besteht,
wobei das mindestens eine Cannabinoid in mikroverkapselter Form, nanoverkapselter Form oder in gefriergetrockneter Form bereitgestellt wird.

2. Kaugummizusammensetzung nach Anspruch 1, wobei das mindestens eine Cannabinoid Cannabidiol, Δ⁹-Tetrahydrocannabinol, Cannabichromen, Cannabigerol, Cannabidivarin oder deren Säuremetaboliten ist.

3. Kaugummizusammensetzung nach Anspruch 1, wobei das mindestens eine Cannabinoid in Kombination mit mindestens einem geeigneten Träger bereitgestellt wird, der aus der Gruppe ausgewählt ist, die aus Zuckeralkohol, Cellulosen, wie zum Beispiel mikrokristalline Cellulosederivate, Dextran, Agarose, Agar, Pektin, Alginat, Xanthan, Chitosan und Stärke besteht.

4. Kaugummizusammensetzung nach Anspruch 1, wobei das mindestens eine Cannabinoid in inneren Hohlräumen innerhalb eines geeigneten festen Trägers bereitgestellt wird.

5. Kaugummizusammensetzung nach Anspruch 1, wobei das mindestens eine Cannabinoid in einem Granulat innerhalb der Gummimatrix bereitgestellt wird.

6. Kaugummizusammensetzung nach Anspruch 1, wobei das mindestens eine Cannabinoid aus natürlichen Quellen oder synthetisch gewonnen wird.

7. Kaugummizusammensetzung nach Anspruch 1, wobei das mindestens eine Opioid ein Opioid-Agonist ist, der aus der Gruppe ausgewählt ist, die aus Morphin, Kodein, Thebain, Hydrocodon, Hydromorphon, Oxycodon, Oxymorphon, Buprenorphin, Fentanyl, Methadon, Pethidin, Levorphanol, Tramadol und Dextropropoxyphen besteht.

8. Kaugummizusammensetzung nach Anspruch 1, wobei das mindestens eine Opioid ein Opioid-Prodrug ist, das aus der Gruppe ausgewählt ist, die aus 6-Monoacetylmorphin, Nicomorphin, Dipropanoylmorphin, Desomorphin, Methyldesorphin, Acetylpropionylmorphin, Dibenzoylmorphin und Diacetyldihydromorphin besteht.

9. Kaugummizusammensetzung nach Anspruch 1, wobei das mindestens eine Opioid in nanoverkapselter oder mikroverkapselter Form bereitgestellt wird.

10. Kaugummizusammensetzung nach Anspruch 1, die ferner mindestens ein Konservierungsmittel und mindestens ein Antioxidans umfasst.

11. Kaugummizusammensetzung nach Anspruch 10, wobei das Konservierungsmittel Zitronensäure ist.

12. Kaugummizusammensetzung nach Anspruch 10, die ferner mindestens einen pharmazeutisch verträglichen Hilfsstoff umfasst, der aus der Gruppe ausgewählt ist, die aus Füllstoffen, Desintegrationsmitteln, Bindemitteln und Gleitmitteln besteht.

13. Kaugummizusammensetzung nach Anspruch 12, die ferner Siliciumdioxid oder Magnesiumstearat umfasst.

14. Kaugummizusammensetzung nach Anspruch 1, die ferner mindestens einen Opioid-Antagonisten umfasst, der aus der Gruppe ausgewählt ist, die aus Naloxonhydrochlorid-Dehydrat und Naltrexonhydrochlorid in einem Gewichtsverhältnis von 4 Opioid : 1 Opioid-Antagonist besteht.

## Revendications

1. Composition de gomme à mâcher comprenant, par rapport au poids total de la composition :
0,1 à 5 % en poids d'au moins un cannabinoïde ;
0,01 à 1 % en poids d'au moins un opioïde ;
au moins 20 % en poids d'une base de gomme ;
5 à 35 % en poids d'au moins un agent tampon choisi dans le groupe consistant en les acétates, les glycinates, les phosphates, les carbonates, les glycérophosphates, les citrates et les borates ;
1 à 10 % en poids d'au moins un agent aromatisant choisi dans le groupe consistant en menthe poivrée, menthe verte, réglisse, cannelle, pastèque, vanille, ananas, pomme et canneberge ;
1 à 65 % en poids d'au moins un agent édulcorant choisi dans le groupe consistant en isomalt, sorbitol, stévia, maltitol et xylitol ; et
au moins un anti-oxydant choisi dans le groupe consistant en palmitate d'ascorbyle et ascorbate de sodium,
où l'au moins un cannabinoïde est fourni sous forme microencapsulée, sous forme nanoencapsulée ou sous forme lyophilisée.

2. Composition de gomme à mâcher selon la revendication 1, où l'au moins un cannabinoïde est le cannabidiol, le Δ⁹-tétrahydrocannabinol, le cannabichromène, le cannabigérol, la cannabidivarine ou leurs métabolites acides.

3. Composition de gomme à mâcher selon la revendication 1, où l'au moins un cannabinoïde est fourni en combinaison avec au moins un support approprié choisi dans le groupe consistant en alcool de sucre, celluloses, telles que les dérivés de cellulose microcristalline, dextran, agarose, agar, pectine, alginate, xanthane, chitosane et amidon.

4. Composition de gomme à mâcher selon la revendication 1, où l'au moins un cannabinoïde est fourni dans des vides internes à l'intérieur d'un support solide approprié.

5. Composition de gomme à mâcher selon la revendication 1, où l'au moins un cannabinoïde est fourni dans un granule à l'intérieur de la matrice de gomme.

6. Composition de gomme à mâcher selon la revendication 1, où l'au moins un cannabinoïde est obtenu à partir de sources naturelles ou synthétiques.

7. Composition de gomme à mâcher selon la revendication 1, où l'au moins un opioïde est un agoniste opioïde choisi dans le groupe consistant en morphine, codéine, thébaïne, hydrocodone, hydromorphone, oxycodone, oxymorphone, buprénorphine, fentanyl, méthadone, péthidine, lévorphanol, tramadol et dextropropoxyphène.

8. Composition de gomme à mâcher selon la revendication 1, où l'au moins un opioïde est un promédicament opioïde choisi dans le groupe consistant en 6-monoacétyl morphine, nicomorphine, dipropanoylmorphine, désomorphine, méthyldésorphine, acétylpropionyl morphine, dibenzoyl morphine et diacétyl dihydromorphine.

9. Composition de gomme à mâcher selon la revendication 1, où l'au moins un opioïde est fourni sous forme nanoencapsulée ou microencapsulée.

10. Composition de gomme à mâcher selon la revendication 1, comprenant en outre au moins un conservateur et au moins un antioxydant.

11. Composition de gomme à mâcher selon la revendication 10, où le conservateur est l'acide citrique.

12. Composition de gomme à mâcher selon la revendication 10, comprenant en outre au moins un excipient pharmaceutiquement acceptable choisi dans le groupe consistant en charges, désintégrants, liants et lubrifiants.

13. Composition de gomme à mâcher selon la revendication 12, comprenant en outre du dioxyde de silicium ou du stéarate de magnésium.

14. Composition de gomme à mâcher selon la revendication 1, comprenant en outre au moins un antagoniste opioïde choisi dans le groupe comprenant le chlorhydrate de naloxone déshydraté et le chlorhydrate de naltrexone à un rapport en poids de 4 opioïde : 1 antagoniste opioïde.
